# EUROPEAN PATENT SPECIFICATION

(11) **EP 2 948 556 B1**
(45) Date of publication and mention of the grant of the patent: **14.06.2017**
(21) Application number: 14711847.5
(22) Date of filing: 24.01.2014
(51) Int. Cl.: C12P 7/56, C12N 9/02

(54) **PROCESS FOR THE SEQUESTRATION OF CARBON DIOXIDE AND THE FERMENTATIVE PRODUCTION OF ORGANIC COMPOUNDS**
VERFAHREN ZUR SEQUESTRIERUNG VON KOHLENDIOXID UND FERMENTATIVEN HERSTELLUNG ORGANISCHER VERBINDUNGEN
PROCÉDÉ POUR LA SÉQUESTRATION DE DIOXYDE DE CARBONE ET LA PRODUCTION PAR FERMENTATION DE COMPOSÉS ORGANIQUES

(30) Priority: 24.01.2013 IT MI20130109
(43) Date of publication of application: 02.12.2015
(73) Proprietor: Consiglio Nazionale Delle Ricerche, 00185 Roma (IT)
(72) Inventor: FONTANA, Angelo, I-83100 Avellino (IT); D'IPPOLITO, Giuliana, I-80128 Napoli (IT); DIPASQUALE, Laura, I-80010 Quarto (IT)
(74) Representative: Cattaneo, Elisabetta
(86) International application number: PCT/IB2014/058519
(87) International publication number: WO 2014/115110

(56) References cited:
- NIELS T ERIKSEN ET AL: "Hydrogen production in anaerobic and microaerobic Thermotoga neapolitana", BIOTECHNOLOGY LETTERS, SPRINGER NETHERLANDS, DORDRECHT, vol. 30, no. 1, 12 September 2007 (2007-09-12), pages 103-109, XP019548758, ISSN: 1573-6776, DOI: 10.1007/S10529-007-9520-5
- D'IPPOLITO G ET AL: "Hydrogen metabolism in the extreme thermophile Thermotoga neapolitana", INTERNATIONAL JOURNAL OF HYDROGEN ENERGY, ELSEVIER SCIENCE PUBLISHERS B.V., BARKING, GB, vol. 35, no. 6, 29 January 2010 (2010-01-29), pages 2290-2295, XP026915128, ISSN: 0360-3199, DOI: 10.1016/J.IJHYDENE.2009.12.044 [retrieved on 2010-01-29]
- SARAH A. MUNRO ET AL: "The fermentation stoichiometry of Thermotoga neapolitana and influence of temperature, oxygen, and pH on hydrogen production", BIOTECHNOLOGY PROGRESS, vol. 25, no. 4, 23 June 2009 (2009-06-23), pages 1035-1042, XP055116930, ISSN: 8756-7938, DOI: 10.1002/btpr.201
- YU JIANG ET AL: "A highly efficient method for liquid and solid cultivation of the anaerobic hyperthermophilic eubacterium Thermotoga maritima", FEMS MICROBIOLOGY LETTERS, vol. 259, no. 2, 1 June 2006 (2006-06-01), pages 254-259, XP055117052, ISSN: 0378-1097, DOI: 10.1111/j.1574-6968.2006.00273.x
- LAURA DIPASQUALE ET AL: "Capnophilic lactic fermentation and hydrogen synthesis by Thermotoga neapolitana: An unexpected deviation from the dark fermentation model", INTERNATIONAL JOURNAL OF HYDROGEN ENERGY, vol. 39, no. 10, 14 February 2014 (2014-02-14), pages 4857-4862, XP055117147, ISSN: 0360-3199, DOI: 10.1016/j.ijhydene.2013.12.183
- FURDUI CRISTINA ET AL: "The role of pyruvate ferredoxin oxidoreductase in pyruvate synthesis during autotrophic growth by the Wood-Ljungdahl pathway", JOURNAL OF BIOLOGICAL CHEMISTRY, AMERICAN SOCIETY FOR BIOCHEMISTRY AND MOLECULAR BIOLOGY, vol. 275, no. 37, 30 June 2000 (2000-06-30), pages 28494-28499, XP002231080, ISSN: 0021-9258, DOI: 10.1074/JBC.M003291200
- TAKESHI IKEDA ET AL: "Enzymatic and electron paramagnetic resonance studies of anabolic pyruvate synthesis by pyruvate: ferredoxin oxidoreductase from Hydrogenobacter?thermophilus", FEBS JOURNAL, vol. 277, no. 2, 15 January 2010 (2010-01-15), pages 501-510, XP055080721, ISSN: 1742-464X, DOI: 10.1111/j.1742-4658.2009.07506.x
- NEUER G ET AL: "The pyruvate: Ferredoxin oxidoreductase in heterocysts of the cyanobacteriuim Anabaena cylindrica", BIOCHIMICA ET BIOPHYSICA ACTA (BBA) - GENERAL SUBJECTS, ELSEVIER, vol. 716, no. 3, 16 June 1982 (1982-06-16) , pages 358-365, XP025787352, ISSN: 0304-4165, DOI: 10.1016/0304-4165(82)90028-9 [retrieved on 1982-06-16]

### SEQUENCE LISTING

<110> CONSIGLIO NAZIONALE DELLE RICERCHE
<120> Process for the sequestration of carbon dioxide and the fermentative
   production of organic compounds
<130> 12139PTWO
<150> ITMI2013A000109
   <151> 2013-01-24
<160> 4
<170> BiSSAP 1.2
<210> 1
   <211> 394
   <212> PRT
   <213> Thermotoga neapolitana
<220>
   <223> PorA subunit YP_002534223.1
<400> 1
   Met Lys Met Glu Arg Val Val Glu Arg Val Ala Val Thr Gly Ala Glu
   1               5                   10                  15
   Ala Val Ala Asn Ala Met Arg Gln Ile Glu Pro Asp Val Val Ala Ala
               20                  25                  30
   Tyr Pro Ile Thr Pro Gln Thr Pro Ile Val Glu Tyr Phe Ala Arg Phe
           35                  40                  45
   Val Ala Asp Gly Ile Val Lys Thr Glu Met Ile Pro Val Glu Ser Glu
       50                  55                  60
   His Ser Ala Met Ser Ala Val Val Gly Ala Ala Ala Ala Gly Ala Arg
   65                  70                  75                  80
   Ala Met Thr Ala Thr Ser Ala Asn Gly Leu Ala Leu Met His Glu Ile
                   85                  90                  95
   Val Tyr Ile Ala Ala Ser Tyr Arg Leu Pro Ile Val Met Pro Val Val
               100                 105                 110
   Asn Arg Ala Leu Ser Gly Pro Ile Asn Ile His Cys Asp His Ser Asp
           115                 120                 125
   Ala Met Ala Glu Arg Asp Ser Gly Trp Ile Gln Leu Phe Ala Glu Thr
       130                 135                 140
   Asn Gln Glu Ala Tyr Asp Phe Thr Ile Leu Ala Val Arg Leu Ala Glu
   145                 150                 155                 160
   His Ser Asp Val Arg Leu Pro Val Met Val Asn Leu Asp Gly Phe Ile
                   165                 170                 175
   Leu Ser His Gly Val Glu Pro Val Glu Phe Tyr Pro Asp Asp Leu Val
               180                 185                 190
   Lys Lys Phe Val Gly Glu Leu Lys Pro Met Tyr Pro Leu Leu Gly Thr
           195                 200                 205
   Glu His Pro Val Thr Trp Gly Pro Leu Asp Leu Tyr Asp Tyr Tyr Phe
       210                 215                 220
   Glu His Lys Arg Gln Gln Ile Glu Ala Met Glu Asn Val Lys Lys Val
   225                 230                 235                 240
   Phe Pro Glu Ile Ala Lys Glu Phe Glu Glu Thr Phe Gly Arg Lys Tyr
                   245                 250                 255
   Trp Phe Val Glu Pro Tyr Lys Leu Asp Asp Ala Glu His Val Met Val
               260                 265                 270
   Ala Leu Gly Ser Thr Asn Ser Thr Ile Lys Tyr Val Val Asp Glu Leu
           275                 280                 285
   Arg Glu Glu Gly His Lys Val Gly Ala Leu Lys Ile Trp Met Phe Arg
       290                 295                 300
   Pro Phe Pro Lys Glu Gln Leu Gln Glu Leu Leu Asn Gly Arg Lys Ser
   305                 310                 315                 320
   Val Ile Val Leu Asp Arg Ala Val Ser Phe Gly Ala Glu Ala Pro Leu
                   325                 330                 335
   Tyr Glu Ala Val Lys Ser Ser Leu Tyr Glu Val Ala Ala Arg Pro Ser
               340                 345                 350
   Leu Gly Ser Tyr Val Tyr Gly Leu Gly Gly Arg Asp Ile Lys Pro Glu
           355                 360                 365
   His Ile Arg Lys Ala Phe Glu Asp Ala Ile Asn Gly Asn Leu Ile Ala
       370                 375                 380
   Asp Glu Gln Arg Tyr Leu Gly Leu Arg Glu
   385                 390
<210> 2
   <211> 324
   <212> PRT
   <213> Thermotoga neapolitana
<220>
   <223> PorB subunit YP_002534222.1
<400> 2
   Met Pro Val Asn Ile Lys Gln Leu Ala Gln Glu Phe Asp Lys Lys Glu
   1               5                   10                  15
   Ile Gly Ile Thr Gln Gly His Arg Leu Cys Pro Gly Cys Gly Ala Pro
               20                  25                  30
   Ile Thr Val Lys Phe Val Met Met Ile Ala Arg His Leu Gly Tyr Glu
           35                  40                  45
   Pro Val Val Gly Leu Ala Thr Gly Cys Leu Glu Val Ser Thr Thr Ile
       50                  55                  60
   Tyr Pro Tyr Thr Ala Trp Ser Val Pro Tyr Ile His Asn Ala Phe Glu
   65                  70                  75                  80
   Asn Val Ala Ala Thr Met Ser Gly Val Glu Thr Ala Tyr Arg Ala Leu
                   85                  90                  95
   Lys Asn Lys Gly Lys Ile Pro Glu Asp Lys Lys Tyr Ala Phe Ile Ala
               100                 105                 110
   Phe Gly Gly Asp Gly Gly Thr Tyr Asp Ile Gly Leu Gln Ser Leu Ser
           115                 120                 125
   Gly Met Leu Glu Arg Gly His Lys Val Leu Tyr Val Leu Tyr Asp Asn
       130                 135                 140
   Glu Gly Tyr Met Asn Thr Gly Asn Gln Arg Ser Gly Ser Thr Pro Pro
   145                 150                 155                 160
   Gly Ser Asp Thr Thr Thr Ala Pro Val Gly Lys Lys Leu Pro Gly Lys
                   165                 170                 175
   Val Gln Leu Lys Lys Asn Ile Val Glu Ile Val Ala Ala His Glu Asn
               180                 185                 190
   Val Tyr Ala Ala Thr Ala Ala Leu Ser Glu Pro Met Asp Phe Phe Ala
           195                 200                 205
   Lys Val Glu Lys Ala Leu Asn Phe Asp Gly Pro Ser Phe Leu Ala Val
       210                 215                 220
   Leu Ser Pro Cys Val Arg Phe Trp Arg Val Asn Asp Asp Lys Thr Val
   225                 230                 235                 240
   Glu Ile Ser Lys Leu Ala Val Glu Thr Lys Tyr Trp Pro Leu Tyr Glu
                   245                 250                 255
   Val Glu Arg Gly Val Tyr Arg Val Thr Arg Lys Pro Arg Gln Phe Lys
               260                 265                 270
   Pro Val Glu Glu Phe Leu Arg Ala Gln Gly Arg Phe Arg Lys Leu Leu
           275                 280                 285
   Ser Arg Pro Asp Ala Lys Glu Ile Ile Asp Glu Leu Gln Glu Tyr Val
       290                 295                 300
   Asp Arg Arg Trp Glu Arg Leu Leu Ala Leu Glu Glu Ala Thr Lys Asp
   305                 310                 315                 320
   Lys Pro Ile Arg
<210> 3
   <211> 199
   <212> PRT
   <213> Thermotoga neapolitana
<220>
   <223> PorC subunit YP_002534225.1
<400> 3
   Met Cys Lys Glu Val Gln Ser Met Pro Val Ala Lys Lys Tyr Phe Glu
   1               5                   10                  15
   Ile Arg Trp His Gly Arg Ala Gly Gln Gly Ala Lys Ser Ala Ser Gln
               20                  25                  30
   Met Leu Ala Glu Ala Ala Leu Glu Ala Gly Lys Phe Val Gln Ala Phe
           35                  40                  45
   Pro Glu Tyr Gly Ala Glu Arg Thr Gly Ala Pro Met Arg Ala Phe Asn
       50                  55                  60
   Arg Ile Gly Asp Glu Tyr Ile Arg Val Arg Ser Ala Ile Glu Asn Pro
   65                  70                  75                  80
   Asp Val Val Val Val Ile Asp Glu Thr Leu Leu Ser Pro Ala Ile Val
                   85                  90                  95
   Glu Gly Leu Ser Glu Asp Gly Ile Leu Leu Val Asn Thr Val Lys Asp
               100                 105                 110
   Phe Glu Phe Val Arg Gln Lys Thr Gly Phe Asn Gly Lys Ile Cys Val
           115                 120                 125
   Val Asp Ala Thr Asp Ile Ala Leu Gln Glu Ile Lys Arg Gly Ile Pro
       130                 135                 140
   Asn Thr Pro Met Leu Gly Ala Leu Val Arg Val Thr Gly Val Val Pro
   145                 150                 155                 160
   Leu Glu Val Ile Glu Lys Arg Ile Glu Lys Met Phe Gly Lys Lys Phe
                   165                 170                 175
   Pro Gln Glu Val Val Glu Ala Asn Lys Arg Ala Leu Arg Arg Gly Tyr
               180                 185                 190
   Glu Glu Val Lys Cys Ser Glu
           195
<210> 4
   <211> 99
   <212> PRT
   <213> Thermotoga neapolitana
<220>
   <223> PorD subunit YP_002534224.1
<400> 4
   Met Ser Leu Lys Ser Trp Lys Glu Ile Pro Ile Gly Gly Val Ile Asp
   1               5                   10                  15
   Lys Pro Gly Thr Ala Arg Glu Tyr Lys Thr Gly Thr Trp Arg Val Met
               20                  25                  30
   Arg Pro Ile Leu His Lys Glu Lys Cys Ile Asp Cys Met Phe Cys Trp
           35                  40                  45
   Leu Tyr Cys Pro Asp Gln Ala Ile Val Gln Glu Gly Gly Ile Met Lys
       50                  55                  60
   Gly Phe Asn Tyr Asp Tyr Cys Lys Gly Cys Gly Leu Cys Ala Asn Val
   65                  70                  75                  80
   Cys Pro Lys Gln Ala Ile Glu Met Arg Pro Glu Thr Glu Phe Leu Gly
                   85                  90                  95
   Glu Glu Gly

## Description

### FIELD OF THE INVENTION

The present invention relates to the use of biological components of anaerobic microorganisms, in particular cultures or enzymes of anaerobic bacteria, as agents for the capture and sequestration of carbon dioxide by reaction with an appropriate organic substrate. The products resulting from this reaction are organic molecules usable as raw materials in industries, such as food, biomedical, cosmetics and zootechnical industries.

### STATE OF THE ART

The current consensus of the scientific community is that the atmospheric concentration of CO₂ will continue to increase in a way that is directly proportional to the consumption of fossil fuels, with significant consequences for the global climate. Clearly, the optimal scenario for the future entails a transition to renewable energy, but, alternatively or in interim strategies, sequestration or attenuation are deemed to be the main objectives to mitigate the risk of greenhouse-induced climate changes.

Eriksen et al "Hydrogen production in anaerobic and microaerobic Thermotoga neapolitana" discloses the production of H₂ from Thermotoga neapolitana. It shows also acetic acid produced as well as minor amounts of lactic acids, but by using N₂ or N₂ replaced with air (6%O₂).

The object of the present invention, therefore, is to provide a process for abating carbon dioxide.

### SUMMARY OF THE INVENTION

The above indicated object has been attained by a process able to capture carbon dioxide and convert it into organic compounds usable as industrial raw materials. Hence, the invention pertains to a process for producing at least one organic compound of formula (I): 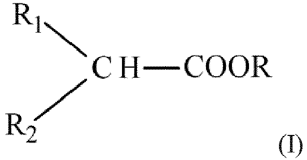 wherein
R is H or alkaline metal,
R₁ is -H, -OH, -O-alkyl, -NH₂, -NH-alkyl, -SH, where alkyl is linear or branched C1-C3,
R₂ is -H, -OH, linear or branched C1-C3 alkyl, or linear or branched C1-C3 alkyl substituted with at least one -OH group or -NH₂ group,
provided that R₁ and R₂ are not simultaneously -H,
comprising the steps of:
i) providing at least one bacterium of the taxonomic Order *Thermotogales* comprising pyruvate:ferredoxin oxidoreductase (PFOR), ferredoxin, acetyl-coenzyme A synthetase, and coenzyme A;
ii) adding an organic substrate;
iii) adding CO₂;
iv) reacting the resulting mixture under anaerobic conditions for at least 6 hours; and
v) separating the at least one organic compound of formula (I) thus obtained.

According to another aspect, the present invention concerns a plant for implementing said process, comprising:
- at least one reactor, provided with inlet for feeding CO₂, inlet for feeding the organic substrate, outlet for collecting the reaction products, and outlet for collecting gas;
- at least one device for separating the reaction products; and
- at least one container for collecting the at least one organic compound of formula (I) separated.

In the present invention, when using the term:
- "organic substrate", what is meant is one or more compounds, preferably formic acid or salts thereof, acetic acid or salts thereof, propionic acid and salts thereof, butyric acid or salts thereof, said one or more compounds being provided in pure form or as an *in situ* metabolic product of simple organic matrices, such as monosaccharides, disaccharides, polysaccharides, proteins, or of complex organic matrices, such as byproducts or waste materials of the food processing industry, of agriculture, or algal biomasses; and
- "CO₂" or "carbon dioxide", what is meant is that said component may be in gaseous form, pure or mixed with other gases, or in the form of an inorganic salt, in solid form or in solution, or in an organic matrix able to release it.

### BRIEF DESCRIPTION OF THE FIGURES

The characteristics and advantages of the present invention will be apparent from the following detailed description, as well as the working examples provided for illustrative and non limiting purposes, and the attached Figures wherein:
- Figure 1 shows the sequence listing of the tetrameric protein PFOR;
- Figure 2 shows the metabolic pathway that, in the bacterium *T. neapolitana,* glucose follows in the presence of CO₂;
- Figure 3 shows (a) the metabolic pathway that, in the bacterium *T. neapolitana,* glucose follows in the presence of CO₂ and (b) the corresponding reactions of sequestration of [¹³C]-CO₂ which take place along said pathway; bold line = bond between two paired carbons;
- Figure 4 shows the gel SDS-PAGE (10%) of the Western Blot test of the expression of the PFOR enzyme in cultures of *T. neapolitana* grown in standard conditions and fed at regular intervals with N₂ (*Tn*N) or CO₂ (*Tn*C);
- Figure 5a shows the glucose consumption rate in cultures of *T. neapolitana* in the presence of glucose 25mM and fed with N₂ or CO₂;
- Figure 5b shows the hydrogen (H₂) yield after 24 hours and after 48 hours of the cultures of *T. neapolitana* in the presence of glucose 25mM and fed with N₂ (*Tn*N) or CO₂ (*TnC*);
- Figure 5c shows the acetic acid and lactic acid yield after 24 hours and after 48 hours of the cultures of *T. neapolitana* in the presence of glucose 25mM and fed with N₂ (*Tn*N) or CO₂ (*Tn*C);
- Figure 5d shows the yield of the products: acetic acid, lactic acid and hydrogen, expressed in terms of the product mol/glucose mol ratio of the cultures of *T. neapolitana* in the presence of glucose 25mM and fed with N₂ (*TnN*), CO₂ (*TnC*) or N₂ *(TnN)* + NaHCO₃ 14mM;
- Figure 6 shows a schematic view of an embodiment of the plant of the invention; 1 = feeding inlet of the substrate for the reaction with carbon dioxide; 2 = feeding inlet of carbon dioxide, either gaseous or in solution; 3 = hydrogen outlet; 4 = reactor; 5 = product outlet;
- Figure 7 shows the ¹³C-NMR spectra of the culture medium of *T. neapolitana* after feeding with ¹³CO₂ or NaH¹³CO₃. (a) fed with ¹³CO₂ (*TnC*); (b) fed with 14 mM NaH¹³CO₃ and N₂; (c) control. * = marked carbon; bold line = bond between two paired carbons; and
- Figure 8 shows the ¹³C-NMR spectra of the culture medium of *T. neapolitana* after feeding with 14 mM NaH¹³CO and different organic substrates. Growing was carried out under standard conditions with insufflation of N₂. Spectra enlargements
- indicated the paired ¹³C carbons. (a) 28 mM 2-¹³C glucose and 14 mM NaH¹³CO₃; (b) 14 mM 2-¹³C sodium pyruvate and 14 mM NaH¹³CO₃; (c) 14 mM 1-¹³C sodium acetate and 14 mM NaH¹³CO₃; (d) control (28 mM glucose and 14 mM NaH¹³CO₃).
- Figure 9 shows the synthesis of lactic acid in cultures of *Thermotoga neapolitana* kept in nitrogen or carbon dioxide atmosphere. In accordance with the mechanism known as Dark Fermentation, glucose metabolism leads to the theoretical synthesis of four moles of hydrogen and two moles of acetic acid (black arrows). The increase of carbon dioxide in the reactor causes the recycling of the acetate (red arrows) with consequent synthesis of lactic acid.

### DETAILED DESCRIPTION OF THE INVENTION

Hence, the invention concerns a process for producing at least one organic compound of formula (I): 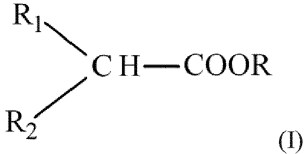 wherein
R is H or alkaline metal,
R₁ is -H, -OH, -O-alkyl, -NH₂, -NH-alkyl, -SH, where alkyl is linear or branched Cl-C3,
R₂ is -H, -OH, linear or branched C1-C3 alkyl, or linear or branched C1-C3 alkyl substituted with at least one -OH group or -NH₂ group, provided that R₁ and R₂ are not simultaneously -H,
comprising the steps of:
i) providing at least one bacterium of the taxonomic Order *Thermotogales* comprising pyruvate:ferredoxin oxidoreductase (PFOR), ferredoxin, acetyl-coenzyme A synthetase, and coenzyme A;
ii) adding an organic substrate;
iii) adding CO₂;
iv) reacting the resulting mixture under anaerobic conditions for at least 6 hours; and
v) separating the at least one organic compound of formula (I) thus obtained.

It was surprisingly found that the cells of said at least one bacterium of the taxonomic Order *Thermotogales,* thermophilic and heterotrophic, act as micro-reactors able to catalyze the reaction between organic substrate and carbon dioxide to produce the organic compound of formula (I), which has low molecular weight, in particular lactic acid, through a direct process without fixation of CO₂ in the cellular biomass. In fact, CO₂ is, for all intents and purposes, captured and used as a reactant in the synthesis of the compound having formula (I), without entering into cell growth mechanisms that are typical, for example, of autotrophic bacteria.

Preferably, said at least one bacterium of the taxonomic Order *Thermotogales* is a bacterium of the genus *Thermotoga,* such as *T. neapolitana, T. naphtophila, T. petrophila, T. maritima, T subterranea, T. elfii, T. lettingae, T. thermarum,* or *Thermosipho,* such as *T. geodei, T. melanesiensis, T. japonicus, T. africanus.*

These bacteria are able to produce hydrogen by anaerobic and non-photosynthetic fermentation (dark fermentation) of carbohydrates. In particular, as shall be seen in the following Examples, a production of H₂ was observed that was very close to the theoretical value of 4 molecules of H₂ per molecule of glucose that enters into the glycolytic process. Total hydrogen production corresponds to approximately 0.25-0.50 m³ of hydrogen gas per kilogram of glucose. The fermentative synthesis of hydrogen is associated with the release of two molecules of acetic acid and two of carbon dioxide per glucose molecule.

In a preferred embodiment, said at least one bacterium of the taxonomic Order *Thermotogales,* is of the species *Thermotoga neapolitana. T. neapolitana* may be isolated from marine sources of sea water.

Preferably, the bacteria *Thermotogales* are provided in an aqueous medium, such as sea water or water enriched with mineral salts, vitamins and similar nutrients.

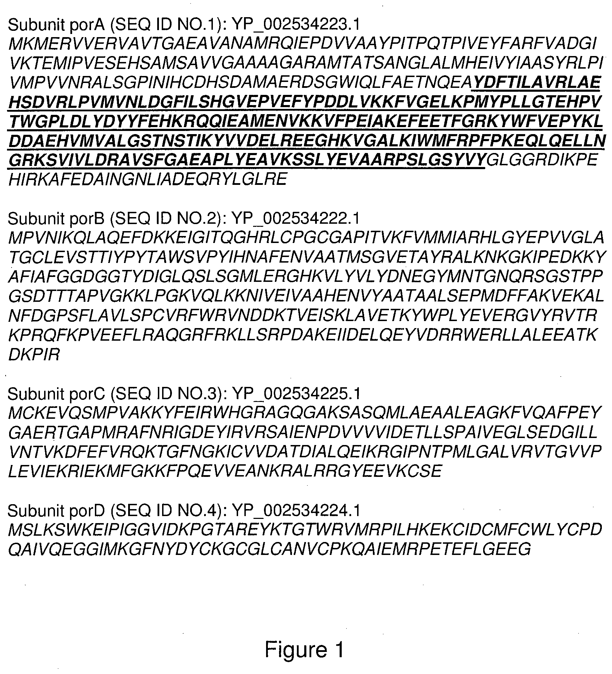

According to a particularly preferred embodiment, said PFOR is a tetrameric protein having access numbers NCBI YP_002534223.1 (SEQ ID NO.1), YP_002534222.1 (SEQ ID NO.2), YP_002534225.1 (SEQ ID NO.3), YP_002534224.1 (SEQ ID NO.4) and it is encoded by the genetic cluster *por BACD* of the bacterium *Thermotoga neapolitana* (Figure 1):
Subunit porA (SEQ ID NO.1): YP_002534223.1
Subunit porB (SEQ ID NO.2): YP_002534222.1
Subunit porC (SEQ ID NO.3): YP_002534225.1
Subunit porD (SEQ ID NO.4): YP_002534224.1

In particular, said at least one bacterium, mainly thanks to the presence of PFOR, and of other reducing agents such as reduced ferredoxin, NADH and NADPH, is able to capture and sequester carbon dioxide, advantageously promoting the synthesis of the at least one organic compound of formula (I), which can then be further utilized as an industrial raw material, for example in the food, biomedical, cosmetic or zootechnical industries.

As will be shown herein below, the supply of CO₂ directly influences the main parameters for the production of said at least one organic compound of formula (I) when the reaction mixture is allowed to react as such.

Advantageously, the carbon dioxide may be an exhaust gas from industrial facilities or from thermoelectric power plants, thereby contributing to the abatement of atmospheric pollution.

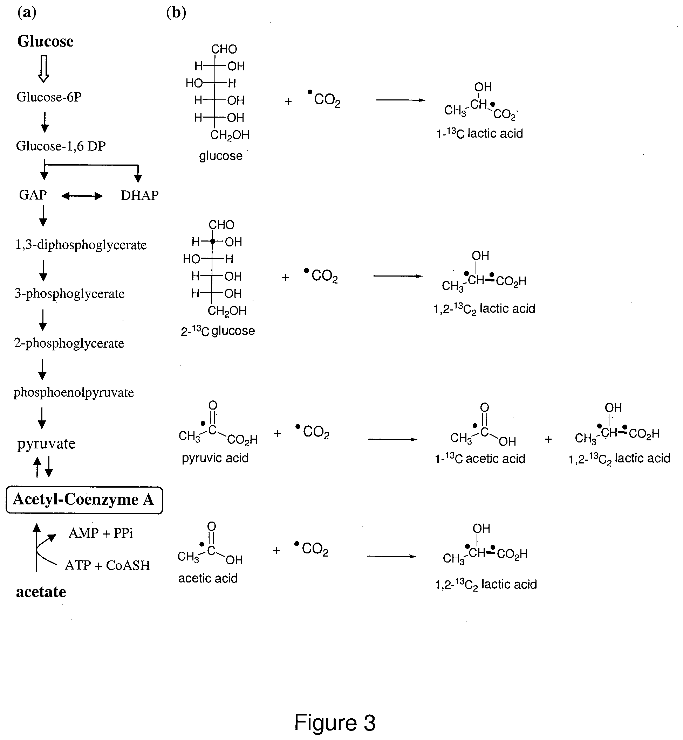

The carbon dioxide can be added, for example in the form of exhaust gas, at the same time, in order to heat the reaction environment, preferably to temperatures between 60 and 80°C. Therefore, according to the invention, the organic substrate, within the bacterial cells, reacts with CO₂ to give the at least one organic compound of formula (I), by direct synthesis when, in the presence of Coenzyme A and acetyl-coenzyme A synthetase, the organic substrate is formic acid or salts thereof, acetic acid or salts thereof, propionic acid and salts thereof, butyric acid or salts thereof, in pure form or passing through the formation of a keto-acid intermediate compound, when the organic substrate is a simple or complex organic matrix comprising a metabolic precursor of formic acid or salts thereof, acetic acid or salts thereof, propionic acid and salts thereof, butyric acid or salts thereof (Figure 3).

In step iv) of the process of the invention, the mixture is allowed to react for at least 6 hours, thus obtaining the at least one organic compound of formula (I).

Preferably, in step iv) of the process, the mixture is allowed to react for 12 to 72 hours, more preferably 20 to 50 hours.

Preferably, said organic substrate comprises a compound selected from formic acid or salts thereof, acetic acid or salts thereof, propionic acid and salts thereof, butyric acid or salts thereof, a monosaccharide, a disaccharide, a polysaccharide, or mixtures thereof.

The term "monosaccharide", means a simple carbohydrate, i.e. one that cannot be decomposed by hydrolysis. Non-substituted monosaccharides have the general formula (CH₂O)ₙ (with n=3, 4, 5, ...). The number of carbon atoms ranges from 3 to 9 for monosaccharides with higher molecular weight, but the most important ones are pentoses (arabinose, ribose, xylose, etc.) and hexoses (glucose, fructose, galactose etc.).

The term "disaccharide" means an acetal formed by two monosaccharides bonded by an acetal bond commonly called O-glycosidic bond. The most important disaccharides are: cellobiose, two molecules of D-glucose with 1-4 β glycosidic bond; maltose, two molecules of α-D-glucose with 1-4 α glycosidic bond; lactose, one molecule of D-glucose and one of D-galactose with 1-4 β glycosidic bond; saccharose, one molecule of glucose and one of fructose with 1-2 α glycosidic bond; trehalose, two molecules of glucose with 1,1 α glycosidic bond.

The term "polysaccharide" means compounds formed by at least 3 monosaccharides. Among the most important are cyclodextrin, melitose, fructo-oligosaccharides, agarose, glycogen, dextran, starch, cellulose, hemicellulose, pectin, amylopectin, amylose, chitin, callose, laminarin, mannan, fucoidan, galactomannan, inulin, dextrin, maltodextrin, glycans and xylans.

Preferably, said organic substrate comprises acetic acid or salts thereof or a metabolic precursor thereof, such as a monosaccharide; more preferably, said at least one metabolic precursor is glucose.

Preferably, said at least one organic compound has formula (I), wherein:
R is H or alkaline metal,
R₁ is -H, -OH, -O-alkyl, where alkyl is linear or branched C1-C3,
R₂ is linear or branched C1-C3 alkyl, or linear or branched C1-C3 alkyl substituted with at least one -OH group or -NH₂ group,

In a preferred embodiment, said at least one organic compound of formula (I) is lactic acid. According to a preferred embodiment, the process of the invention comprises the steps of:
i) providing bacterium *Thermotoga neapolitana* wherein pyruvate:ferredoxin oxidoreductase (PFOR) is a tetrameric protein having SEQ ID NO: 1, SEQ ID NO: 2, SEQ ID NO: 3 and SEQ ID NO: 4;
ii) adding an organic substrate comprising acetic acid or salts thereof, or glucose, and obtaining acetyl-CoA;
iii) adding CO₂;
iv) reacting the resulting mixture in anaerobic, non-photosynthetic conditions for at least 6 hours; and
v) separating lactic acid thus obtained.

Said glucose can be in pure form or in organic matrices that contain it.

It should be understood that all the possible combinations of the preferred aspects of the process, as described above, are also similarly preferred, and hence described.

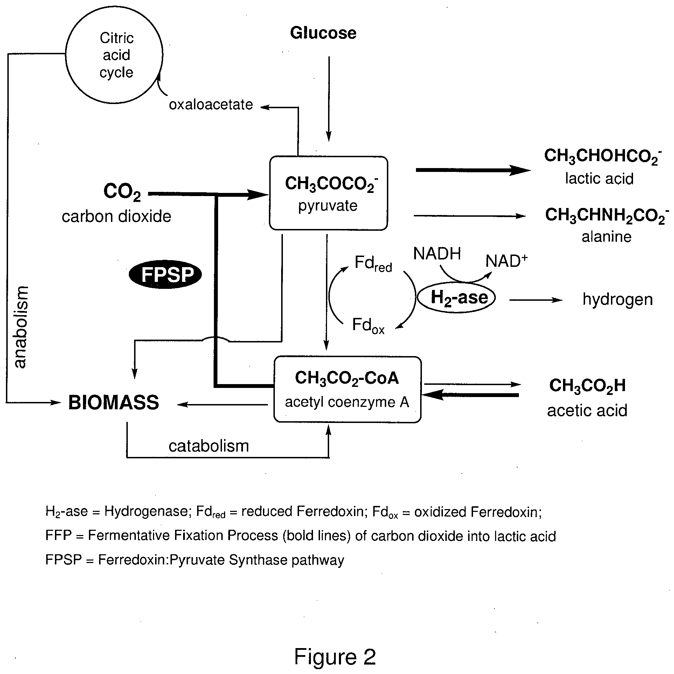

As illustrated in Figure 2, *T. neapolitana* has a new biochemical pathway for the direct synthesis of pyruvate from acetyl coenzyme A (Ac-CoA) and carbon dioxide (CO₂). The pathway is activated by elevated levels of CO₂ and it represents the first case of carbon dioxide sequestration by coupling with an exogenous substrate (i.e., the organic substrate employed in the present process) to form a final product, i.e., organic compound of formula (I), in particular, lactic acid, directly released outside the cells. This process corresponds to a type of fermentative and heterotrophic assimilation.

As shall be better illustrated by the following Example, in this case, the use of acetic acid and CO₂ is in molar agreement with the synthesis of lactic acid as a result of the substitution of the oxidative decarboxylation of the pyruvate with a NADH-dependent reduction of the pyruvate to lactic acid. However, the process shows an apparently inconsistent carbon balance, which is due to the ability of the anaerobic culture to sequester carbon dioxide through mechanisms that are not yet altogether clear.

It should be pointed out that, as shown in Figures 2 and 3, the production of H₂ by dark fermentation is associated to the release of organic acid, such as acetic acid, in the culture medium as final products of the metabolic transformation of the sugars. The accumulation of these metabolites inhibits the synthesis of hydrogen, but the process is easily restored by adjusting the pH, thus allowing to obtain the complete consumption of the sugars.

Moreover, the high partial pressure of H₂ tends to inhibit the production of H₂, deflecting the metabolic pathway towards derivatives of pyruvic acid such as lactic acid or alanine.

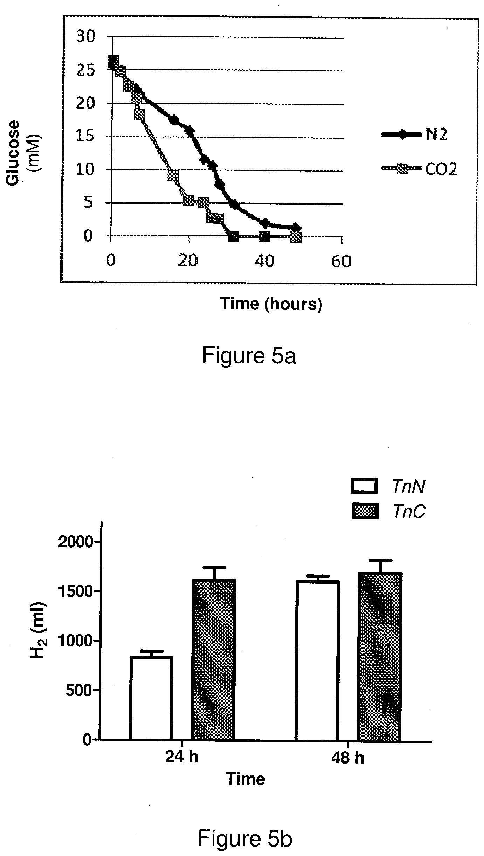

This inhibitory effect was surprisingly overcome, in the present invention, by feeding CO₂ into the reaction environment. In this way, it was observed that the synthesis of hydrogen and acetic acid frequently occurs simultaneously with the production of other organic acids, among which lactic acid (LA) is by far the most abundant. Analysis of the cultures grown in the presence of N₂ alone, as a control, or with CO₂ showed that glucose consumption and the growth rate were substantially different, as described in the Example that follows. In fact, cells grown with CO₂ exhibited significantly higher growth rates and glucose consumption (Figure 5a). At the same time, an increase in lactic acid was also observed, but no reduction in hydrogen yield. This was systematically due to the synthesis of the new pool of pyruvate deriving from acetate recycling (Figure 2). The overall process includes carboxylation of the acetate by carbon dioxide to give pyruvate and then lactic acid, according to the following reaction scheme:

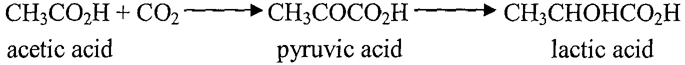

Bicarbonate or other carbonated salts are possible alternatives to the feeding of pure CO₂, although lower efficiency is observed in the specific case of PFOR encoded by the genetic cluster *porBACD* of the bacterium *Thermotoga neapolitana,* probably because this bacterium does not possess specific mechanisms for carbon dioxide concentration in cells and hence it is possible for CO₂ in pure form to be able to penetrate into the cells through passive mechanisms that instead are more difficult in the case of bicarbonates and carbonates.

According to a further aspect, the present invention concerns a plant for implementing the aforementioned process, comprising:
- at least one reactor, provided with inlet for feeding CO₂, inlet for feeding the organic substrate, outlet for collecting the reaction products, and outlet for collecting gas;
- at least one device for separating the reaction products; and
- at least one container for collecting the at least one organic compound of formula (I) separated.

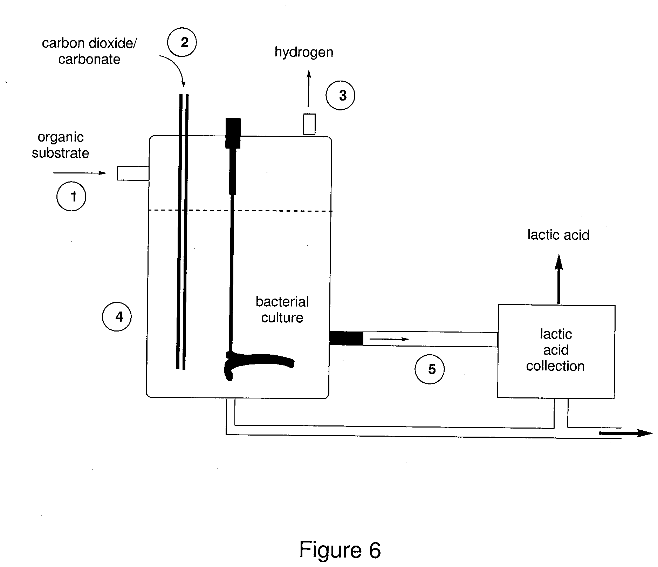

In Figure 6, an embodiment of said plant is schematically shown.

It should to be understood that all the aspects identified as preferred and advantageous for the process of the invention are to be also, analogously, deemed preferred and advantageous for the production plant and the related uses.

Working examples of the present invention are provided for illustrative purposes.

### Example 1.

### Production of lactic acid by the process of the present invention

Microorganisms, media and culture conditions - An anaerobic culture of bacterium *Thermotoga neapolitana* (DSM 4359) was maintained in *Tn* medium supplemented with 25 mM (0.5% wt/v) of glucose and 0.4% (wt/v) of yeast extract/tryptone. The *Tn* medium contained (g/L): NaCl 10; KCl 0.1; MgCl₂ • 6 H₂O 0.2; NH₄Cl 1.0; K₂HPO₄ 0.3; KH₂PO₄ 0.3; CaCl₂ • 2 H₂O 0.1; cysteine-HCl 1.0; yeast extract 2.0; tryptone 2.0; glucose 5.0; resazurin (redox marker) 0.001; 1 L distilled H₂O. Before sterilization, the pH was adjusted to 8.0 with 1 M NaOH at room temperature. After sterilization, the medium was then supplemented with 10 mL/L of both vitamins (sterilized by filtration) that solutions of trace elements (as per the commercial product 'Medium 141. METHANOGENIUM MEDIUM' by DSMZ). The excess oxygen was removed by heating the batch reactors, at the same time making oxygen-free gas flowing in them until the solution became colorless. The bacteria (25 mL) had grown overnight at 80°C without stirring and subsequently used to inoculate (1.5 mL, 6% v/v) flasks closed with fluid-tight stoppers (total volume 120 mL).

The bacterium *T neapolitana* had also grown in a 3.8 liter fermenter with a working volume of 1 liter and a head space of 2.8 liters. The cultures were maintained under continuous stirring at 250 rpm at 80°C in the medium described above. The cultures were regularly fed with 30 ml/min of CO₂ or N₂ (control). The fermenters were inoculated with a culture in logarithmic phase (6% v/v) and transferred from the flasks prepared as described above. The pH of the culture was adjusted to 7.5 with 1 M NaOH at ambient temperature and fed with N₂, unless otherwise specified. For experiments with bicarbonate, sodium salt was added (5 mM, 14 mM, 20 mM, 40 mM, 60 mM) under standard conditions (25 mM glucose and 0.4% wt/v yeast extract/tryptone) and the cultures were fed with N₂. Cell growth was determined by optical density (OD) at 540 nm (UV/Vis DU 730 spectrometer, Beckman Coulter). All culture transfers and samplings were carried out with sterile syringes and needles.

Chemical analyses - Acetic acid and lactic acid in cultures of *T. neapolitana* were determined by means of ¹H-NMR with spectrophotometer at 600 MHz (Bruker Avance 400) equipped with Cryoprobe®, using triethylamine hydrochloride (TMA) 3.8 M as an internal standard. The measures of the gases (H₂ and CO₂) were carried out by means of gas-chromatography on an instrument (Focus GC, Thermo Scientific) equipped with a thermal conductivity detector (TCD), integrated to a 3 m packed column (HayeSep Q), and N₂ as gas carrier and reference. The samples were quantified by means of calibration curves made through pure gases. In the culture medium, dissolved CO₂ and bicarbonate were quantified by TDC gas-chromatography after acidification with HCl 6N (2 mL). Glucose concentration was determined with the method of dinitrosalicylic acid calibrated on the standard 2 g/L glucose solution. Protein concentration was determined by Bradford test, using a bovine serum albumin solution (BSA, 1 g/L) as a standard. The measurements were carried out on the supernatants after centrifugation of microbial cultures at 10,000 rpm for 30 min at 5°C.

Carbon 13 (¹³C) experiments - Under standard conditions, the anaerobic cultures of *T*. *neapolitana* in 25 ml of *Tn* medium had been bubbled with a flow of nitrogen that carried ¹³CO₂ deriving from the reaction of ¹³C marked barium carbonate (Ba¹³CO₃) with HCl 1N. A glucose-free medium was used for experiments with 25 mM 2-¹³C glucose or 25 mM 2-¹³C glucose/14 mM NaH¹³CO₃.

The other experiments were carried out in 120-ml sealed flasks under standard conditions with whole medium supplemented with one of the following components: (4.7 mM, 14 mM and 58.3 mM) NaH¹³CO₃; (1.5 mM, 3.5 mM, 7 mM, 14 mM and 28 mM) 2-¹³C-sodium pyruvate/14 mM NaH¹³CO₃; (10 mM and 20 mM) 1-¹³C-sodium acetate/14 mM NaH¹³CO₃. The incubations were carried out in triplicate at 80°C for 72 h with regular feeding of N₂. Sterile NaOH 1M was used to buffer the medium at pH 7.5. After centrifuging, the fermentation products were analyzed directly in samples of the medium by means of NMR (¹H, ¹³C- e ¹³C-¹³C COSY). The supernatants from 0.5 ml culture samples were diluted with 15% D₂O/CD₃OD (1:1 v/v). Hydrogen was measured from the head space of the sealed flasks by TCD gas-chromatography. The marked compounds were supplied by the Cambridge Isotope Laboratories (USA).

Western Blot Analysis - Under standard medium conditions, the bacteria were grown in 3L fermenters for 72h. The cultures were regularly fed with 5 mL CO₂ for 10 minutes every 6 h. The culture means containing an equivalent cell density were collected at every interval, i.e. 6h, 12h, 24h, 30h, 48h and 72h. The cells were gathered by centrifuging at 10,000 rpm for 30 min at 5°C. The samples were suspended in a lysis buffer (50 mM Tris-HCl pH 8.0, 5 mM phenylmethanesulfonyl fluoride) and homogenized by ultrasonication (20 KHz, 8 cycles of 5 min) (Ultrasonic Processor XL). After centrifuging at 3000 rpm at 5°C for 10 min, the opalescent supernatant was clarified by centrifuging at 20,000 rpm for 1 h at 5°C and the resulting material was dialyzed for a long time against 50 mM Tris-HCl pH 8.0 at the same temperature before being loaded onto SDS-PAGE gel (10% acrylamide in separator gel, 5% acrylamide in packing gel, Tris-glycine (pH 8.3) - 1.0 g/L SDS as running buffer). The molecular weight of 250-10 kDa were calculated by means of Precision Plus Protein WesternC Standards (Bio-Rad). The protein bands were colored with 0.1% Coomassie brilliant blue R-250 and de-colored with a mixture of distilled water/methanol/acetic acid (5:4:1 v/v/v). The proteins were transferred by electroblotting on a PVDF Hybond-P membrane (Amersham) and measured using a primary antibody produced in rabbit (1:10000) (PRIMM Srl, Milano, Italy) which recognizes the porA subunit of the PFOR (149 - 359 aa) of *T. neapolitana* (access number NCBI YP_002534223.1), as shown in Figure 1 (underlined sequence portion). The linked antibody was detected by anti-rabbit IgG (whole molecule)-alkaline phosphatase (Sigma Aldrich) as secondary antibody (1:15000) and detected by means of 50 mg/mL 5-bromo-4-chloro-3-indolyl phosphate (BCIP) and 50 mg/mL nitrotetrazolium blue chloride (NBT) (Sigma Aldrich). The blots were repeated three times.

### RESULTS

It was observed that, when the reaction mixture is allowed to react as such, the feeding of CO₂ on distinct cultures of *T, neapolitana* (*TnC*) directly influences the main growth parameters, e.g. growth rate, sugar consumption, H₂ production rate and yield.

Instead, when the reaction mixture is buffered with NaOH, the feeding of CO₂ on distinct cultures of *T. neapolitana* (*TnC*) leads to the acceleration of the fermentation process with the complete consumption of the sugar in the first 24 hours, whereas after 48 hours no substantial differences were observed between the buffered mixtures and the non-buffered mixtures. (see Figures 5a and 5b)

On the basis of sugar consumption, the hydrogen production of the cultures fed with CO₂ was substantially completed in 24 hours, although total production was independent of culture conditions (1612 ml in *Tn*C and 1699 ml in *Tn*N after 48 h). The concentrations of NaHC0₃ (from 5 to 20 mM) gave the same results without apparent variations in glucose consumption and hydrogen production compared to the *Tn*N cultures that were buffered with NaOH 1N. This suggested that the effect on the fermentation process was not due to the direct metabolism of CO₂ but rather to the capability of this gas or of its inorganic salts to maintain the average pH (between 6.0 and 6.5) at more suitable conditions for cell vitality. Also remarkable was the fact that greater quantities of sodium bicarbonate (40 mM and more) negatively influenced bacterial growth, thus reducing the metabolic processes.

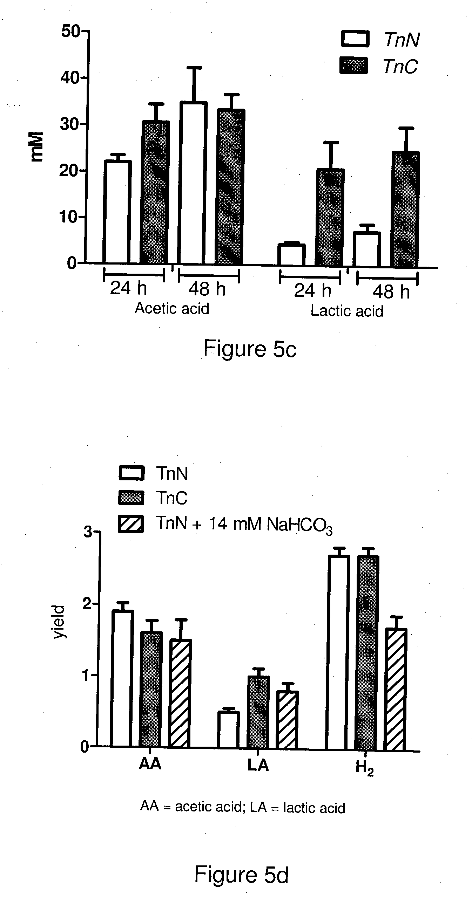

Since pyruvate was obtained by glycolysis from the fermentation of the sugar in *T*. *neapolitana,* introducing glucose marked with ¹³C, a rapid and complete marking of the pyruvate and of the other glycolysis intermediates was consequently observed. (Figure 3). Hydrogen was produced by non-photosynthetic fermentation (dark fermentation (DF)) by means of ferredoxin:NADH-dependent hydrogenase, which transfers electrons to the protons releasing molecular hydrogen. The process was associated to the conversion, which produces NADH, of pyruvate into acetic acid (AA), so the levels of acetic acid were linearly proportional to hydrogen production. On the other hand, the process was negatively influenced by other metabolic reactions that reduced the availability of pyruvate or NADH (e.g. the fermentation of the sugar to lactic acid (LA)). As shown in Figure 5c, the fermentation of acetic acid was comparable in the cultures in *TnN* and *TnC* and it was substantially in agreement with the volumetric production of H₂. Moreover, the feeding of CO₂ (*Tn*C) caused a drastic increase in the levels of lactic acid that, in fact, were found to be approximately five times higher than those in nitrogen atmosphere (*Tn*N)*.* The analysis of the synthesis of the fermentation products in relation to sugar consumption (yield of the products) showed that the yields of acetic acid and hydrogen had not changed in either of the two evaluated systems (Table 1 below). However, the LA/AA yield ratio increased threefold (from 0.27 to 0.66) from *Tn*N to *TnC,* as a consequence of a sharp increase in the production of lactic acid promoted by CO₂ independently of the fermentation of acetic acid.

**Table 1. Production of organic acid from cultures of T. neapolitana supplemented with glucose and fed with N₂ (TnN) or CO₂ (TnC)**

| **Culture** | **Acetic acid (mM)** | | **Lactic acid (mM)** | | **Acetic acid (mM)/ Lactic acid (mM)** | |
|---|---|---|---|---|---|---|
| | **24 h** | **48 h** | **24 h** | **48 h** | **24 h** | **48 h** |
| *Tn*N | 22.1±2.6 | 34.9±13.0 | 4.5±1.0 | 7.4±2.9 | 5.1 | 4.9 |
| *Tn*C | 30.6±6.8 | 33.4±5.8 | 20.9±10.2 | 24.9±9.1 | 1.7 | 1.6 |

*T. neapolitana* tolerates sodium bicarbonate (NaHCO₃) in concentrations between 5 mM and 20 mM, with 14 mM being the preferred concentration to produce AA (18.2 ± 6.4 mM), LA (18.2 ± 0.4 mM) and H₂ (886 mL/h). Above 20 mM, the salt significantly diminished bacterial fermentation that was nearly suppressed at concentrations above 40 mM (Tables 2 and 3 below). The addition of 14 mM NaHCO₃ induced an increase of lactic fermentation compared to the standard *Tn*N conditions, but at the same time it reduced the evolution of hydrogen. However, the boost on LA production due to NaHCO₃ was significantly lesser than the one observed with the feeding of CO₂ (24.9 ± 9.1 mM). In spite of the evident variation of the biochemical synthesis of hydrogen, and lactic acid in different experimental conditions (Figure 5d), analysis of the conversion of glucose revealed that the yields of AA (1.75 ± 0.13 with NaHCO₃, 1.82 ± 0.09 with CO₂, 1.92 ± 0.21 in standard conditions) were not substantially influenced by the bicarbonate 14 mM.

**Table 2. Production of H₂ from fermentation of glucose 25 mM through cultures of T. neapolitana fed with N₂ and supplemented with sodium bicarbonate or 14 mM sodium bicarbonate / carbonic anhydrase (TnN + CA).**

| **Culture** | **Sugar consumption %** | | **H₂ production rate, mL/L/h** | | **H₂ production yield, mol H₂/mol glu** | |
|---|---|---|---|---|---|---|
| | **24 h** | **48 h** | **24 h** | **48 h** | **24 h** | **48 h** |
| *Tn*N + 14 mM NaHCO₃ | 56.2 ± 6.3 | 90.6 ± 2.7 | 30.1 ± 2.4 | 18.5 ± 0.9 | 2.0 ± 0.07 | 1.7 ± 0.07 |
| *TnN* + 20 mM NaHCO₃ | 53.9 ± 0.3 | 82.8 ± 3.5 | 16.2 ± 5.2 | 14.5 ± 5.8 | 1.1 ± 0.3 | 1.0 ± 0.2 |
| *TnN* + 40 mM NaHCO₃ | 10.0 ± 2.1 | 22.0 ± 1.9 | 16.1 ± 2.7 | 8.3 ± 1.3 | 2.9 ± 0.4 | 2.7 ± 0.6 |
| *TnN* + CA | 31.7 ± 3.7 | 75.0 ± 6.4 | 31.0 ± 3.1 | 25.3 ± 2.9 | 2.9 ± 0.3 | 2.0 ± 0.05 |

**Table 3. Production of organic acids from fermentation of glucose 25 mM through cultures of T. neapolitana fed with N₂ and supplemented with sodium bicarbonate or 14 mM sodium bicarbonate / carbonic anhydrase (TnN + CA).**

| **Culture** | **Acetic Acid (mM)** | | **Lactic Acid (mM)** | | **Acetic Acid (mM)/ Lactic Acid (mM)** | |
|---|---|---|---|---|---|---|
| | **24 h** | **48 h** | **24 h** | **48 h** | **24 h** | **48 h** |
| *Tn*N + NaHCO₃ 14 mM | 24.6 ± 3.2 | 32.1 ± 4.9 | 11.7 ± 0.7 | 18.2 ± 0.4 | 2.1 | 1.8 |
| *Tn*N + NaHCO₃ 20 mM | 28.8 ± 6.0 | 39.4 ± 1.2 | 5.5 ± 3.7 | 9.8 ± 4.7 | 6.3 | 4.6 |
| *TnN* + NaHCO₃ 40 mM | 12.5 ± 1.2 | 13.0 ± 4.3 | 0.7 ± 1.7 | 0.7 ± 1.5 | 16.7 | 18.6 |
| *Tn*N+BCA | 23.9 | 42.4 | 0.7 | 3.0 | 33.5 | 14.2 |

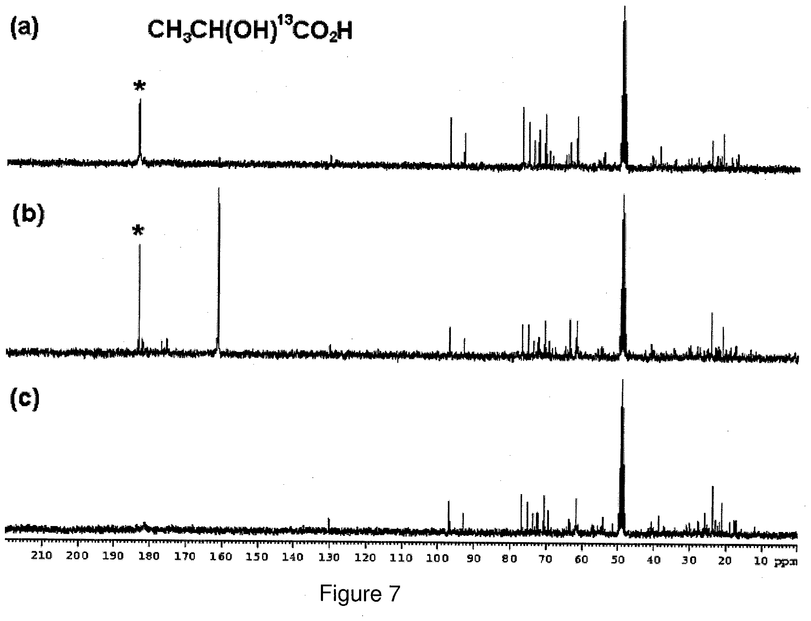

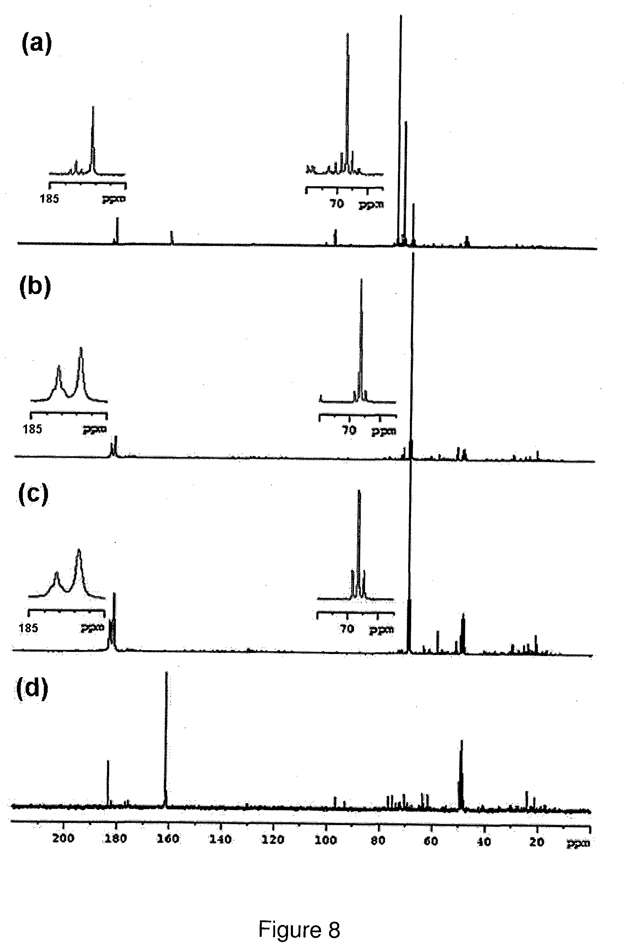

The absorption of carbon dioxide in the cells was tested by bubbling, in the bacterial culture, a flow of ¹³CO₂ generated by the reaction of ¹³C marked barium carbonate (Ba¹³CO₃) with HCl 1M. Nitrogen was used as a gas carrier. The ¹³C NMR spectra of the fermentation broth of these cultures showed the clear incorporation of carbon dioxide marked at the C-1 (183.3 ppm) of lactic acid (see NMR in Figure 7a, whilst Figure 7c shows the NMR of the control). The specific enrichment was further corroborated by HMBC experiments that showed the correlation of the signal of the ¹³C at 183.3 ppm with the methyl and methylene protons of lactic acid (respectively at 1.33 and 4.09 ppm). Similarly, the growth of *T. neapolitana* on glucose supplemented with ¹³C marked sodium bicarbonate (NaH¹³CO₃) 14 mM, as expected, led to the specific production of 1-¹³C lactic acid (see NMR in Figure 7b). Glycolysis splits glucose in the middle, converting the carbon positions 1, 2, and 3 (and 6, 5, and 4) respectively into methyl, carbonyl, and carboxyl carbons of the pyruvate. Consequently, in view of the predominance of the Embden-Meyerhof pathway, the fermentation of 2-¹³C₂-glucose produced only 1-¹³C₂-acetate and 2-¹³C-lactate in *T. neapolitana* under standard conditions. By contrast, the growth of the thermophilic bacteria on *Tn* supplemented with 25 mM 2-¹³C-glucose and 14 mM NaH¹³CO₃ gave the expected 1-¹³C acetate, together with 1,2-¹³C₂ lactate (see NMR in Figure 8a). The synthesis of this latter compound was unequivocal because the vicinal carbons at 69.3 ppm (C-2) and 183.3 ppm (C-1) of lactic acid resonated as doublets (*J* = *54.6* Hz) by homonuclear coupling. This proved that the bond between C-1 and C2 of lactic acid was newly formed by reaction of the carbon dioxide and of a C₂ unit deriving from glucose, similarly acetate or its equivalent form acetyl coenzyme A. No isotopic scrambling phenomena were observed in the fermentation products of *T. neapolitana,* nor was there any marking of other carbons of acetate and lactate, produced by the bacterium. Consistently with the marking of the lactic acid, a double marking was also found at C1 (176.5 ppm, *J =* 54.6) and C-2 (51.5 ppm, *J =* 54.6 Hz) of alanine. Since this amino acid derives from the direct transamination of pyruvate, the overall results indicated the specific loss of the carbonyl carbon of the pyruvate presumably in the exchange reaction with the carbon dioxide present in the environment.

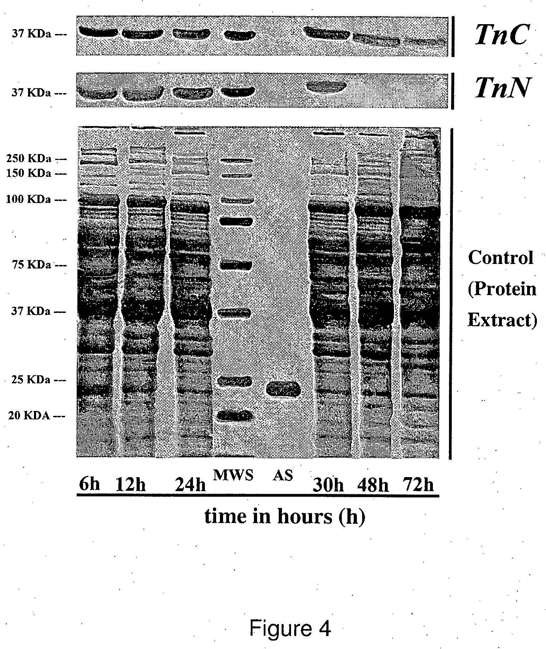

The cells of *T. neapolitana* were also able to capture 2-¹³C-pyruvate added to the *Tn* medium. In fact, the metabolism of this precursor led to 1-¹³C-acetate and to a lesser extent to 2-¹³C-lactate. On the other hand, experiments with 2-¹³C-pyruvate 28 mM and NaH¹³CO₃ 14 mM obtained doubly enriched lactic acid and alanine (see NMR in Figure 8b), which was the same result obtained with the experiments whereby marked glucose and carbon dioxide were fed, as described above. The metabolism of the pyruvate was, in fact, surprising in a heterotrophic bacterium although it could be due to the partial restoration of the endogenous pool of this acid by the outer substrate. However, the synthesis of 1,2-¹³C₂ lactic acid necessarily required the decarboxylation of the pyruvate to acetyl-CoA followed by the reductive transfer of carbon dioxide to this latter substrate and the new synthesis of 1,2-¹³C₂ pyruvic acid (see Figure 3). In vitro, the reaction is catalyzed by the pyruvate:ferredoxin oxidoreductase (PFOR). The use of reduced ferredoxin, whose potential redox is close to that of pyruvate as an oxidant, has the potential to make the coupling reaction possible, although PFOR is known only in reverse reactions from pyruvate to acetyl-CoA and CO₂ in physiological conditions. To test the synthetic function of PFOR in *T. neapolitana,* the bacterium had been grown under standard conditions on glucose supplemented with 1-¹³C acetate 20 mM and NaH¹³CO₃ 14 mM. The NMR analysis of the culture medium revealed the diagnostic doublets (*J*= 54.6) at 183.3 and 69.3 ppm for C-1 and C-2 of the doubly marked lactic acid (see NMR in Figure 8c). This proved the coupling of acetate and CO₂ and the synthesis of lactic acid from the reduction of a transient pool of newly produced pyruvate or biochemically equivalent forms. The PFOR of *T. neapolitana* is a heterotetrameric complex consisting of four peptide chains called porA, porB, porC and porD (NCBI database accession numbers: YP_002534223.1, YP_002534222.1, YP_002534225.1, YP_002534224.1). *porA, porB, porC,* and *porD* are clustered in the genome of *T. neapolitan* and show high homology to genes having similar functions in other members of the order *Thermotogales.* For the sake of practicality, reference is .expressly made to the reaction of the pyruvate synthase (*Tn*PS), but enzyme is generically referred to as PFOR. To verify the variation of *Tn*PS during bacterial growth and in relation to the presence of CO₂, PFOR levels were analyzed in cultures of *T. neapolitana* by means of polyclonal antibodies against the antigenic peptide 149 - 359 (AS) of porA of the tetrameric complex. In accordance with the recognized catalytic function of effecting the decarboxylation of pyruvate to acetyl-CoA, it was found that the signal of PFOR disappeared with the consumption of glucose in cultures under N₂ after 36 h (Figure 4). This result was in line with a positive feedback of glucose on the expression of the protein. On the contrary, the immunoblots of the cell extracts from cultures under CO₂ clearly showed a persistent presence of PFOR throughout the experiment (72 h) in spite of the rapid consumption of sugar after only 24 h. No significant differences were observed in the levels of the protein before and after this point, thus suggesting that CO₂ stimulates the *ex novo* expression of PFOR, but this regulation is not correlated to glucose metabolism and the expression of PFOR is independently induced by the two factors.

### Example 2.

### Capture and sequestration of carbon dioxide and production of lactic acid

The ability of *Thermotoga neapolitana* to absorb exogenous acetate was tested by administering 2 uCi of [2-¹⁴C]- sodium acetate to bacteria held in 25 ml cultures and grown in sealed 120 ml bottles containing 28 mm (125 mg) of glucose. After the addition of the inoculum at 6 % (v/v), the cultures were incubated at 80°C and pH was buffered to 7.5 with NaOH (1M). After 48 hours and regular sparging with CO₂, the bacterial cells were collected by centrifuging at 13,000 rpm for 10 min. Each pellet was resuspended in 10 g/L of NaCl and washed three times with the same solution before lyophilization. Supernatant (0,5 mL), wash solutions (1 ml) and cell pellets (20 mg of dry weight /ml MeOH) were diluted with OptiPhase "Hisafe" 2 (Perkin Elmer) to a final volume of 10 ml and radioactivity was measured with a scintillation analyzer (Tri - Carb 2100 TR, Perkin Elmer). As shown in Table 4 below, the measurements indicated that less than 5% of radioactivity (4980±446 dpm/mg of biomass) was actually incorporated in the biomass of the bacterial cells, thus confirming that the reductive carboxylation mechanism did not entail an actual fixation of the substrate in the biomass.

**Table 4. Carbon assimilation in the biomasses of Thermotoga neapolitana grown in nitrogen atmosphere (TnN2) or CO₂ atmosphere (TnCO2)**

| | Growth conditions of the bacterial strain | Administered radioactivity | Recovered radioactivity | Percentage of incorporation |
|---|---|---|---|---|
| | | µCi | dpm | % |
| [2-¹⁴C]-sodium | *Tn*N2 | 2'354×10³ | 48'095±4'364 | 5.0 |
| acetate | *Tn*CO2 | 2'354×10³ | 48'801±2'882 | 5.1 |

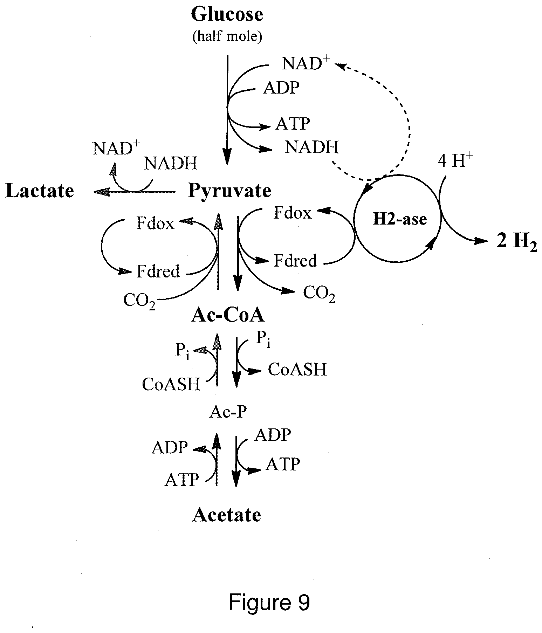

As illustrated in Figure 9 for the synthesis of lactic acid, the mechanism for the synthesis of organic compounds of formula (I) in bacterium *Thermotoga neapolitana* implies the capture and sequestration of environmental carbon dioxide in the compound of formula (I). To estimate the quantitative efficiency of the process, 14 mM NaH¹³CO₃ and 20 mM of 1-¹³C-sodium acetate were added to cultures of *T. neapolitana* in standard conditions. The bacteria were kept for 3 days at 80°C. After centrifuging, the organic acids were purified from the culture medium with a GracePure™ anion-exchange column. The column was washed with 2% (w/v) of NaOH (10 mL) and then equilibrated in H₂O (10 mL). After elution with 10 ml of bidistilled H₂O, the organic acids were recovered by means of a solution of 1M HCl (1.5 mL) and H₂O (1.5 mL). The fraction containing organic acids was extracted three times with equal quantities of ethyl acetate. After evaporation of the organic solvent, the upper fractions were dissolved in methanol at a final concentration of 10 mg/ml and directly analyzed with a LC-MS equipped with a quadrupole-TOF analyzer using a source with electrospray ionization (ESI) in negative ion mode.

The analysis of the ratio between the masses of the isotopologues of the lactic acid produced by the cultures showed the following composition: M⁻ = *m*/*z* 88.9657 for ¹²CH₃¹²CH(OH)¹²CO₂⁻, 67.7%; (M+1)⁻ = *m*/*z* 89.9701 for ¹²CH₃¹³CH(OH)¹²CO₂⁻ and ¹²CH₃¹²CH(OH)¹³CO₂⁻, 24.9%; (M+2)⁻ = *m*/*z* 90.9755 for ¹²CH₃¹³CH(OH)¹³CO₂⁻, 7.4%).

This composition indicated the presence of approximately 0.05 mmol of doubly marked product out of a total of 0.637 mmol of total lactic acid produced by the bacterium. Considering that the cultures were administered 0.35 mmol of marked sodium bicarbonate, the measurement established that nearly 15% of the inorganic carbon had been captured and sequestered in the lactic acid. Obviously, this value established only the quantity of lactic acid produced starting from acetate and CO₂ that were marked and bonded directly to form the doubly marked metabolite (peak M+2), but it underestimated the actual contribution of the exogenous precursors because it did not take into consideration the union of the marked products with unmarked acetate and CO₂. As a good approximation, this latter combination is responsible for the mass of lactic acid with value M+1. Since this isotopologue of lactic acid corresponded to approximately 25% of the entire quantity of organic acid produced by the bacterial cultures, it can be estimated that approximately 40% of the marked bicarbonate administered to the cultures was captured and sequestered in lactic acid through the process of the present invention.

## Claims

1. A process for producing at least one organic compound of formula (I): 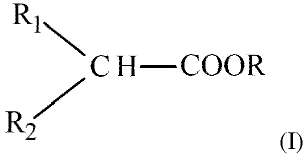 wherein
R is H or alkaline metal,
R₁ is -H, -OH, -O-alkyl, -NH₂, -NH-alkyl, -SH, where alkyl is linear or branched C1-C3,
R₂ is -H, -OH, linear or branched C1-C3 alkyl, or linear or branched C1-C3 alkyl substituted with at least one -OH group or -NH₂ group,
provided that R₁ and R₂ are not simultaneously -H,
comprising the steps of:
i) providing at least one bacterium of the taxonomic Order *Thermotogales* comprising pyruvate:ferredoxin oxidoreductase (PFOR), ferredoxin, acetyl-coenzyme A synthetase, and coenzyme A;
ii) adding an organic substrate;
iii) adding CO₂;
iv) reacting the resulting mixture under anaerobic conditions for at least 6 hours; and
v) separating the at least one organic compound of formula (I) thus obtained.

2. The process of claim 1, wherein said at least one bacterium is of the genus *Thermotoga* or *Thermosipho.*

3. The process of claim 2, wherein said at least one bacterium is of the species *Thermotoga neapolitana* or *Thermotoga Maritima.*

4. The process of claim 3, wherein said PFOR is a tetrameric protein having SEQ ID NO.1, SEQ ID NO.2, SEQ ID NO.3, SEQ ID NO.4

5. The process of anyone of claims 1-4, wherein said at least one organic compound has formula (I), wherein:
R is H or alkaline metal,
R₁ is -H, -OH, -O-alkyl, where alkyl is linear or branched C1-C3,
R₂ is linear or branched C1-C3 alkyl, or linear or branched C1-C3 alkyl substituted with at least one -OH group or -NH₂ group.

6. The process of claim 5, wherein said at least one organic compound of formula (I) is lactic acid.

7. The process of any one of claims 1-6, wherein said organic substrate comprises a compound selected from formic acid or salts thereof, acetic acid or salts thereof, propionic acid and salts thereof, butyric acid or salts thereof, a monosaccharide, a disaccharide, a polysaccharide, or mixtures thereof.

8. The process of claim 7, wherein said organic substrate comprises acetic acid or salts thereof, or one monosaccharide.

9. The process of claim 8, wherein said one monosaccharide is glucose.

10. The process of any one of claims 4-9, comprising the steps of:
i) providing bacterium *Thermotoga neapolitana* wherein pyruvate:ferredoxin oxidoreductase (PFOR) is as defined in claim 4;
ii) adding an organic substrate comprising acetic acid or salts thereof, or glucose, to obtain acetyl-CoA;
iii) adding CO₂;
iv) reacting the resulting mixture under anaerobic and non-photosynthetic conditions for at least 6 hours; and
v) separating lactic acid thus obtained.

## Patentansprüche

1. Verfahren zur Herstellung mindestens einer organischen Verbindung mit der Formel (I): 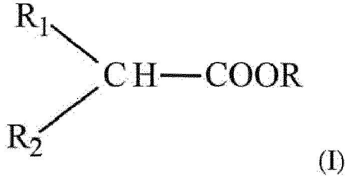 wobei
R H oder Alkalimetall ist,
R₁ -H, -OH, -O-Alkyl, -NH₂, -NH-Alkyl, -SH ist, wobei Alkyl lineares oder verzweigtes C1-C3 ist,
P₂ -H, -OH, lineares oder verzweigtes C1-C3-Alkyl oder lineares oder verzweigtes C1-C3-Alkyl substituiert mit mindestens einer -OH-Gruppe oder -NH₂-Gruppe ist,
vorausgesetzt, dass R₁ und R₂ nicht gleichzeitig -H sind,
umfassend die Schritte:
i) Bereitstellen mindestens eines Bakteriums der taxonomischen Ordnung *Thermotogales* umfassend Pyruvat:Ferredoxin-Oxidoreduktase (PFOR), Ferredoxin, Acetyl-Coenzym-A Synthetase und Coenzym A;
ii) Zugabe eines organischen Substrats;
iii) Zugabe von CO₂;
iv) Reagieren der resultierenden Mischung unter anaeroben Bedingungen für mindestens 6 Stunden; und
v) Abtrennen der mindestens einen so erhaltenen organischen Verbindung mit der Formel (I).

2. Verfahren nach Anspruch 1, wobei das mindestens eine Bakterium von der Gattung *Thermotoga* oder *Thermosipho* ist.

3. Verfahren nach Anspruch 2, wobei das mindestens eine Bakterium von der Spezies *Thermotoga neapolitana* oder *Thermotoga Maritima* ist.

4. Verfahren nach Anspruch 3, wobei das PFOR ein tetrameres Protein mit SEQ ID NO. 1, SEQ ID NO. 2, SEQ ID NO. 3, SEQ ID NO. 4 ist.

5. Verfahren nach einem der Ansprüche 1 bis 4, wobei die mindestens eine organische Verbindung die Formel (I) aufweist, wobei:
R H oder Alkalimetall ist,
R₁ -H, -OH, -O-Alkyl ist, wobei Alkyl lineares oder verzweigtes C1-C3 ist,
R₂ lineares oder verzweigtes C1-C3-Alkyl oder lineares oder verzweigtes C1-C3-Alkyl substituiert mit mindestens einer -OH-Gruppe oder -NH₂-Gruppe ist.

6. Verfahren nach Anspruch 5, wobei die mindestens eine organische Verbindung mit der Formel (I) Milchsäure ist.

7. Verfahren nach einem der Ansprüche 1 bis 6, wobei das organische Substrat eine Verbindung ausgewählt aus Ameisensäure oder ihren Salzen, Essigsäure oder ihren Salzen, Propionsäure und ihren Salzen, Buttersäure oder ihren Salzen, einem Monosaccharid, einem Disaccharid, einem Polysaccharid oder ihren Mischungen umfasst.

8. Verfahren nach Anspruch 7, wobei das organische Substrat Essigsäure oder ihre Salze oder ein Monosaccharid umfasst.

9. Verfahren nach Anspruch 8, wobei das eine Monosaccharid Glucose ist.

10. Verfahren nach einem der Ansprüche 4-9, umfassend die Schritte:
i) Bereitstellen des Bakteriums *Thermotoga neapolitana,* wobei Pyruvat:Ferredoxin-Oxidoreduktase (PFOR) wie in Anspruch 4 definiert ist;
ii) Zugabe eines organischen Substrats, das Essigsäure oder ihre Salze oder Glucose umfasst, um Acetyl-CoA zu erhalten;
iii) Zugabe von CO₂;
iv) Reagieren der resultierenden Mischung unter anaeroben und nichtphotosynthetischen Bedingungen für mindestens 6 Stunden; und
v) Abtrennen der so erhaltenen Milchsäure.

## Revendications

1. Procédé de production d'au moins un composé organique de formule (I) : 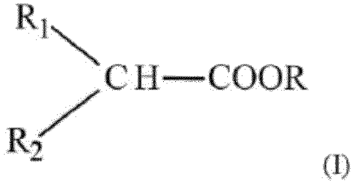 dans laquelle
R représente H ou un métal alcalin,
R₁ représente -H, un groupe -OH, -O-alkyle, -NH₂,-NH-alkyle, -SH, où le groupe alkyle est en C₁ à C₃ linéaire ou ramifié,
R₂ représente -H, un groupe -OH, alkyle en C₁ à C₃ linéaire ou ramifié, ou alkyle en C₁ à C₃ linéaire ou ramifié substitué par au moins un groupe -OH ou un groupe -NH2,
à condition que R₁ et R₂ ne représentent pas simultanément -H,
comprenant les étapes suivantes :
i) la fourniture d'au moins une bactérie de l'ordre taxonomique des *Thermotogales* comprenant la pyruvate-ferrédoxine oxydoréductase (PFOR), la ferrédoxine, l'acétyl-coenzyme A synthétase, et le coenzyme A ;
ii) l'addition d'un substrat organique ;
iii) l'addition de CO₂ ;
iv) la réaction du mélange résultant dans des conditions anaérobies pendant au moins 6 heures ; et
v) la séparation de l'au moins un composé organique de formule (I) ainsi obtenu.

2. Procédé selon la revendication 1, dans lequel ladite au moins une bactérie est du genre *Thermotoga* ou *Thermosipho.*

3. Procédé selon la revendication 2, dans lequel ladite au moins une bactérie est de l'espèce *Thermotoga neapolitana* ou *Thermotoga maritima.*

4. Procédé selon la revendication 3, dans lequel ladite PFOR est une protéine tétramère comportant SEQ ID NO : 1, SEQ ID NO : 2, SEQ ID NO : 3, SEQ ID NO : 4.

5. Procédé selon l'une quelconque des revendications 1 à 4, dans lequel ledit au moins un composé organique a la formule (I), dans laquelle :
R représente H ou un métal alcalin,
R₁ représente -H, un groupe -OH, -O-alkyle, où le groupe alkyle est en C₁ à C₃ linéaire ou ramifié,
R₂ représente un groupe alkyle en C₁ à C₃ linéaire ou ramifié, ou alkyle en C₁ à C₃ linéaire ou ramifié substitué par au moins un groupe -OH ou un groupe -NH₂.

6. Procédé selon la revendication 5, dans lequel ledit au moins un composé organique de formule (I) est l'acide lactique.

7. Procédé selon l'une quelconque des revendications 1 à 6, dans lequel ledit substrat organique comprend un composé choisi parmi l'acide formique ou ses sels, l'acide acétique ou ses sels, l'acide propionique et ses sels, l'acide butyrique ou ses sels, un monosaccharide, un disaccharide, un polysaccharide, ou leurs mélanges.

8. Procédé selon la revendication 7, dans lequel ledit substrat organique comprend de l'acide acétique ou ses sels, ou un monosaccharide.

9. Procédé selon la revendication 8, dans lequel ledit au moins un monosaccharide est le glucose.

10. Procédé selon l'une quelconque des revendications 4 à 9, comprenant les étapes suivantes :
i) la fourniture de la bactérie *Thermotoga neapolitana* dans laquelle la pyruvate-ferrédoxine oxydoréductase (PFOR) est telle que définie dans la revendication 4 ;
ii) l'addition d'un substrat organique comprenant de l'acide acétique ou ses sels, ou du glucose, pour obtenir de l'acétyl-CoA ;
iii) l'addition de CO₂ ;
iv) la réaction du mélange résultant dans des conditions anaérobies et non photosynthétiques pendant au moins 6 heures ; et
v) la séparation de l'acide lactique ainsi obtenu.
